# EUROPÄISCHE PATENTSCHRIFT

(11) **EP 0 589 945 B1**
(45) Veröffentlichungstag und Bekanntmachung des Hinweises auf die Patenterteilung: **10.01.1996**
(21) Anmeldenummer: 92911714.1
(22) Anmeldetag: 16.06.1992
(51) Int. Cl.: A61N 1/20, A61N 1/04

(54) **VORRICHTUNG ZUR BEHANDLUNG VON ENTZÜNDLICHEN, SICH IM ANFANGSSTADIUM BEFINDLICHEN HAUTVERÄNDERUNGEN**
DEVICE FOR TREATING INFLAMMATORY SKIN CHANGES IN THE INITIAL STAGE
DISPOSITIF DE TRAITEMENT D'AFFECTIONS INFLAMMATOIRES DE LA PEAU A LEUR STADE INITIAL

(30) Priorität: 18.06.1991 DE 4120517
(43) Veröffentlichungstag der Anmeldung: 06.04.1994
(73) Patentinhaber: COURAGE + KHAZAKA ELECTRONIC GmbH, D-50829 Köln (DE)
(72) Erfinder: KLEDITSCH, Bernhard, D-1080 Berlin (DE); KHAZAKA, Gabriel, D-5000 Köln 40 (DE)
(74) Vertreter: Sternagel, Hans-Günther, Dr.
(86) Internationale Anmeldenummer: EP9201361
(87) Internationale Veröffentlichungsnummer: WO9222349

(56) Entgegenhaltungen:
- EP-A- 0 158 336
- DE-A- 186 177
- DE-A- 2 304 533
- DE-A- 3 719 353
- FR-A- 1 450 733
- FR-A- 2 266 882
- GB-A- 2 115 700

## Beschreibung

Die vorliegende Erfindung betrifft eine Vorrichtung zur Behandlung, von entzündlichen sich im Anfangsstadium befindlichen Hautveränderungen mit Hilfe von elektrischem Gleichstrom.

Geräte, die zur therapeutischen und kosmetischen Behandlung von Hautpartien elektrischen Gleichstrom anwenden, sind prinzipiell bekannt. So ist im Gebrauchsmuster DE-G 90 17 597.2 ein Gerät für die Iontophorese, insbesondere für die kosmetische Behandlung von Gesichtspartien mit Gleichstromquelle, flüssigkeitsbenetzter Behandlungselektrode und Handelektrode, wobei die Behandlungselektrode sowie die Handelektrode elektrisch mit der Gleichstromquelle verbunden sind. An der Behandlungselektrode werden Wirkstoffe gleichnamiger Ladungen, die in wässriger Lösung vorliegen oder als Salbe auf die Haut aufgetragen worden sind, durch Anlegung von Gleichspannung in die Haut eingeschleust.

In GB-A-2 064 178 ist eine elektrische Versorgungseinheit für ein Gerät zur Silberionentheraphie beschrieben. Dabei wird eine positiv polarisierte Silberelektrode direkt an der zu behandelnden Gewebepartie des Patienten angebracht. Die Gegenelektrode ist an einer anderen Stelle des Körpers befestigt. Durch Einschalten des Gleichstromes werden an der silberhaltigen als Anode geschaltete Elektrode Silberionen freigesetzt, die das zu behandelnde Gewebe bis zu einer Tiefe von 1 cm durchdringen können und deren bakterizide Wirkung in der Behandlung ausgenutzt wird.

Andere Geräte deren Wirkung nicht auf das Einschleusen von Wirkstoffen in das zu behandelnde Gewebe beruhen, sind ebenfalls bekannt. So ist in GB-A-2 181 056 ein Gerät zur Behandlung von entzündlichen Hautveränderungen, insbesondere im Mundraum beschrieben. Das Gerät besteht aus einem Gehäuse, das eine Gleichstromquelle, z.B. eine Batterie sowie eine Behandlungselektrode enthält. Die Behandlungselektrode kann entweder über einen Elektrodenträger direkt mit dem Gehäuse verbunden sein; dabei wirkt dann ein Clip als Gegenelektrode, der über ein flexibles Kabel an die Stromversorgung im Gehäuse angeschlossen ist und z.B. am Ohrläppchen des Patienten angebracht werden kann. Alternativ dazu kann die Behandlungselektrode über ein Kabel mit der Gleichstromquelle im Gehäuse verbunden sein, dann wirkt das Gehäuse selbst, das der Patient in der Hand hält, als Gegenelektrode.

Dieses Gerät zeichnet sich durch eine geringe Bedienungsfreundlichkeit, insbesondere bei der Eigenanwendung durch den Patienten aus. Außerdem ist mit diesem Gerät nur eine sehr punktuelle Behandlung von erkrankten Hautregionen in Abhängigkeit des begrenzten Elektrodenradiuses möglich.

Aus der schweizerischen Patentschrift Nr. 65 020 ist eine Vorrichtung zur galvanischen Körperbehandlung bekannt, die ein Gehäuse mit darin untergebrachter Gleichstromquelle und ein Elektrodenträger, auf dem mindestens zwei entgegengesetzt gepolte Elektroden angeordnet sind, die elektrisch mit der Gleichstromquelle verbunden sind, enthält. Der Elektrodenträger ist dabei fest mit dem Gehäuse verbunden. Die beiden Elektroden können verschiedene Formen annehmen, wobei aber Elektrode und Gegenelektrode grundsätzlich parallel zueinander angeordnet sind, wodurch der zu behandelnde Hautbereich nur von zwei Seiten durch die Elektroden flankiert ist.

GB-A-21 15 700 beschreibt eine Vorrichtung zur Behandlung von Schmerzen mit einer Behandlungselektrode, die auf einer Feder in Längsrichtung beweglich in dem Gehäuse gelagert ist. Die Gegenelektrode umschreibt dabei konzentrison das Gehäuse im Griffteil.

DE-A-37 19 353 offenbart einen elektrischen Stimulator für Nerven, dessen mit Strom-Konstanthalter ausgestatteter Stimulatorstromkreis mittels einer Haut-Klebeelektrode und einer elektrisch-leitenden, aber bis an die Soltze heran auf der Außenseite isolierten Punktiernadel-Elektrode an den Patienten aniegbar ist, wobei ein Stromstärken-Vorwähler mit Stromstärkenzeiger zur Einstellung konstanter Stromstärken zur den Punktier- und Nervensuchvorgang und ein Stromstärkenanzeiger vorgesehen sind.

Aufgabe der vorliegenden Erfindung ist es daher eine Vorrichtung zur Behandlung von entzündlichen sich im Anfangsstadium befindlichen Hautveränderungen zu schaffen, mit der die Nachteile des genannten Standes der Technik vermieden werden. Darüberhinaus soll die Vorrichtung handlich und einfach zu bedienen sein, damit der Patient sie jederseit mit sich führen und auch sich selbst behandeln kann.

Diese Aufgabe wird durch eine Vorrichtung zur Behandlung von entzündlichen, sich im Anfangsstadium befindlichen Hautveränderungen, die ein Gehäuse mit darin untergebrachter Gleichstromquelle und einen Elektrodenträger, auf dem mindestens zwei entgegengesetzt gepolte Elektroden angeordnet sind, die elektrisch mit der Gleichstromquelle verbunden sind, enthält, dadurch gekennzeichnet, daß eine stiftförmige Elektrode von einer oder mehreren Gegenelektroden konzentrisch umgeben ist, wobei der Elektrodenträger in einem zylindrischen Gehäuseteil beweglich auf einer Feder gelagert ist und die innere Stiftelektrode innerhalb des Elektrodenträgers auf einer Feder gelagert ist, deren Rückstellkraft geringer ist als die der ersten Feder, so daß die Elektrode und die Gegenelektrode im Betriebszustand der Vorrichtung auf der zu behandelnden Hautregion aufliegen.

Als Gleichstromquelle dient vorzugsweise eine Blockbatterie, wodurch der Patient das Gerät überall hin mit sich führen kann. Bei einer stationären Behandlung ist es allerdings auch möglich, das Gerät über ein Netzteil direkt an das Stromnetz anzuschließen.

Zusätzlich kann die Vorrichtung eine Schalt- und Regeleinheit mit Kontrolleinrichtung zur überprüfung des Ladezustandes der Batterie und des Betriebszustandes der Vorrichtung sowie eine Einstelleinrichtung für die Betriebsspannung oder den Betriebsstrom aufweisen. Eine geeignete Kontrolleinrichtung kann z.B. eine Trio LED Signalleuchte sein, die bei Betätigung des Batterieprüfschalters den Ladezustand der Batterie anzeigt.

Bei ausreichender Batterieladung leuchtet dann die Trio LED Signalleuchte in einer Farbe, z.B. rot, auf. Gleichzeitig dient die Trio LED Signalleuchte auch als Kontrollanzeige für den Betriebszustand der Vorrichtung. Bei geschlossenem Stromkreis leuchten die Signallampe in einer anderen Farbe, z.B. grün, auf, wobei die Helligkeit in Abhänigkeit des über die Elektroden fließenden Stromes zunimmt.

Alternativ können auch zwei von einander unabhängige Kontrolleuchten oder auch akustische Signalgeber verwendet werden.

Die Wahl der geeigneten Betriebsspannung der Vorrichtung hängt stark von der Empfindlichkeit der zu behandelnden Hautpartie ab. Daher ist eine Einstelleinrichtung für die Betriebsspannung vorgesehen, die zum einen einen Wechselschalter sein kann, mit dem die Spannung auf 9 oder 18 Volt vorgewählt werden kann. Alternativ ist auch eine stufenlose Einstellung der Betriebsspannung zwischen 3 und 18 Volt oder des Betriebsstromes möglich.

Die Elektroden der erfindungsgemäßen Vorrichtung sollten aus einem korrosionsbeständigen Material bestehen, das sich leicht sterilisieren und desinfizieren läßt. Als besonders geeignet haben sich Edelstahl, Edelmetalle sowie Legierungen aus Edelmetallen erwiesen.

Der Kernpunkt der vorliegenden Erfindung liegt in der speziellen Anordnung der Elektroden zu einander, nach der eine Stiftelektrode von einer oder mehreren Gegenelektroden konzentrisch umgeben sind. Diese konzentrische Anordnung von Elektrode und Gegenelektrode hat den Vorteil, daß das zu behandelnde Hautareal vollständig zwischen Anode und Kathode eingeschlossen ist, wodurch der gesamte Bereich von Strom durchflossen wird.

Diese Elektrodenanordnung kann durch verschiedene Ausführungsformen realisiert werden.

In einer bevorzugten Ausführungsform enthält die Vorrichtung zwei Elektroden, wobei eine Elektrode aus einem geraden Stift und die dazu parallel angeordnete Gegenelektrode aus einem Draht besteht, dessen rechtwinklig abgebogenes Teilstück als Drahtschleife ausgebildet ist, die die erste Elektrode konzentrisch umgibt. Die Drahtschleife kann dabei unterschiedlichste Geometrien, wie z.B. die Form eines Kreises, einer Ellipse oder eines Polygons mit 3, 4, 5 oder 6 Ecken annehmen. Der Durchmesser des Kreises oder eines Außenkreises der die Ellipse oder das Polygon umschließt, kann entsprechend der Größe der zu behandelnden Hautregion variiert werden. Als zweckmäßig hat sich ein Durchmesser im Bereich zwischen 5 und 15 mm, vorzugsweise zwischen 8 und 10 mm erwiesen. In der bevorzugten Ausführungsform hat die Drahtschleife die Form eines Kreises, wobei die Spitze der Stiftelektrode und der Drahtring der Gegenelektrode in einer gemeinsamen Ebene liegen.

Alternativ kann auch die erfindungsgemäße Elektrodenanordnung dadurch erreicht werden, daß die Vorrichtung zwei Elektroden enthält, wobei eine Elektrode aus einem geraden Stift besteht, die von einer zylindrischen Gegenelektrode konzentrisch umgeben ist.

Hier hat sich ebenfalls ein Durchmesser zwischen 5 und 15 mm für die zylindrische Elektrode als zweckmäßig erwiesen.

Alternativ kann die Vorrichtung aber auch mehrere stiftförmige Elektroden enthalten, wobei eine Elektrode zentral auf dem Elektrodenträger angeordnet ist und von drei bis acht Gegenelektroden, die die Ecken eines Polygons bilden, konzentrisch umgeben ist. Vorzugsweise umgeben vier oder sechs Elektroden die innere Stiftelektrode. Der Abstand zwischen den äußeren und der inneren Stiftelektrode liegt im Bereich zwischen 5 und 15 mm.

Die bisher beschriebenen Ausführungsformen der erfindungsgemäßen Vorrichtung ermöglichen in Abhängigkeit des Abstandes zwischen der zentralen Elektrode und der diese umgebende Gegenelektrode(n) die Behandlung eines räumlich begrenzten Hautbereiches. Für die Behandlung größerer Hautareale kann die Vorrichtung entsprechend modifiziert werden.

In einer bevorzugten Ausführungsform der erfindungsgemäßen Vorrichtung ist der Elektrodenträger in einem zylindrischen Gehäuseteil angeordnet, das entweder in das Gehäuse, das die Gleichstromquelle enthält, integriert ist oder in Form eines Griffels ausgebildet ist und über ein flexibles Kabel mit dem ersten Gehäuse elektrisch verbunden ist. Der Elektrodenträger ist in dem zylindrischen Gehäuseteil in Längsrichtung beweglich auf einer Feder gelagert und kann gegen die Rückstellkraft der Feder in das Gehäuse hineingeschoben werden, wodurch ein Schalter, der den Stromkreis zu den Elektroden schließt, betätigt wird. Zusätzlich ist die innere Stiftelektrode innerhalb des Elektrodenträgers auf einer Feder gelagert, deren Rückstellkraft geringer ist als die der ersten Feder für den gesamten Elektrodenträger. In diesem Falle ragt die Spitze der zentralen Elektrode über den diese umgebenden konzentrischen Elektrodenring unabhängig davon, ob er durch eine Drahtschleife, einen Zylinder oder eine Vielzahl von stiftförmigen Gegenelektroden gebildet wird, hinaus.

Beim Aufbringen der Elektroden auf die zu behandelnde Hautstelle berührt daher die zentrale Stiftelektrode zuerst die Haut und wird dann gegen die Rückstellkraft der Feder auf der sie im Elektrodenträger gelagert ist, eingedrückt bis auch die Gegenelektrode(n) Kontakt zur Haut hat (haben). Dadurch ist der Abstand zwischen Zentralelektrode und Gegenelektrode variabel und kann sich Hautumebenheiten anpassen. Durch festeres Aufdrücken der Elektroden auf die zu behandelnden Hautstelle wird der Elektrodenträger gegen die Rückstellkraft der ersten Feder in das zylindrische Gehäuseteil hineingeschoben, wodurch ein Schalter betätigt wird, der den Stromkreis zu den Elektroden schließt. Die Lagerung der zentralen Elektrode auf einer Feder geringen Rückstellkraft gewährleistet auch, daß die Kraft mit der die zentrale Elektrode auf die Hautpartie gedrückt wird, deutlich geringer ist als die Kraft mit der die äußere Elektrode(n) auf der Haut aufliegt. Dies ist insbesondere deshalb wichtig, weil die zentrale Elektrode eine geringere Auflagefläche hat, als die sie umgebende(n) Gegenelektrode(n). Dadurch wird der Druck der zentralen Elektrode auf die zu behandelnde Hautstelle reduziert, was insbesondere bei schmerzhaften Hautveränderungen einen zusätzlichen Behandlungskomfort für den Patienten bedeutet. Zusätzlich kann die Vorrichtung nach dieser bevorzugten Ausführungsform einen Schalter zur Umpolung der Elektroden enthalten, der in die Schalt- und Regeleinheit integriert ist.

Das zylindrische Gehäuseteil ist zusätzlich so ausgebildet, daß der Elektrodenträger einfach ausgebaut werden kann und somit leicht gegen Elektrodenträger mit anderen Elektrodenformen oder Abmessungen ausgetauscht oder desinfiziert und sterilisiert werden kann.

Mit der oben beschriebenen Vorrichtung lassen sich sich in beginnender Entzündung (seröse Phase) befindliche Hautbereiche behandeln. Beispiele hierfür sind Herpes labialis, Solitäre Effloressenzen der Neurodermatis, Insektenstiche.

Ein bestehender Duckreiz verschwindet dabei sofort nach der Stromapplikation. Dies kann z.B. in der Allergologie nach intrakutaner Quaddlung einer Substanz, wodurch generell ein starker Duckreiz hervorgerufen wird, angewandt werden.

Bei der Behandlung einer betroffenen Hautregion werden folgende Schritte durchlaufen.

Zuerst wird der Ladezustand der Batterie überprüft und in Abhängigkeit der zu behandelnden Hautregion die Betriebsspannung des Gerätes vorgewählt. Danach wird die zu behandelnde Hautregion intensiv mit einer Elektrolytlösung angefeuchtet, um einen Stromfluß zwischen Elektrode und Gegenelektrode zu ermöglichen.

Geeignete Elektrolytlösungen sind z.B. physiologische Kochsalzlösung, Speichel oder auch Wasser. Danach werden die Elektroden auf die angefeuchtete Hautstelle aufgelegt und der Stromfluß eingeschaltet. Die Behandlung wird fortgesetzt bis sich ein erstes Kribbeln an der zu behandelnden Hautstelle bemerkbar macht.

Ohne eine vollständige Theorie für die Wirkungsweise einer Gleichstrombehandlung angeben zu wollen, sei darauf hingewiesen, daß die Änderung des pH-Wertes um die Elektroden infolge von Elektrolyse der Elektrolytlösung dabei eine Rolle spielt.

Diese pH-Wertänderung tritt nicht nur auf der Oberfläche der Haut auf, sondern läßt sich auch innerhalb der Hautschicht beobachten. Diese pH-Wertänderung verhindert ein weiteres Ausbreiten der Entzündungserreger, dies erklärt auch, warum diese Behandlungsmethode insbesondere bei Entzündung im Anfangsstadium sehr erfolgreich angewendet werden kann.

Ein Vorteil der erfindungsgemäßen Vorrichtung liegt auch in den geringen Außenmaßen und in der einfachen Bedienung.

So kann z.B. ein Patient, der für Herpes labiales empfindlich ist, das Gerät stets mit sich führen und sofort beim ersten spürbaren Kribbeln und beginnendem Spannungsgefühl der betroffenen Haut, unabhängig davon, wo er sich gerade befindet, sofort mit der Behandlung beginnen.

Das Spannungsgefühl und die damit einhergehende Schmerzempfindung läßt sofort nach und die Erkrankung bildet sich innerhalb weniger Stunden vollständig zurück. Dem Patienten bleiben die Bläschen und Schorfbildung auf der Herpes labiales-Wunde sowie die unangenehmen Folgen der

Bewegungseinschränkung der Lippe sowie die Gefahr der Wundheilungsverzögerung durch das Aufplatzen oder Einreißen des Schorfes, aber auch eine mögliche Sekundärinfektion erspart.

Die unterschiedlichen Ausführungsformen der vorliegenden Erfindung werden nun anhand von Zeichnungen näher erläutert.
**Figur 1** zeigt die bevorzugte Ausführungsform der vorliegenden Erfindung in schematischer Darstellung.
**Figur 2** zeigt den Elektrodenträger mit der bevorzugten Elektrodenanordnung im Querschnitt von oben.
**Figur 3** zeigt die Anordnung des Elektrodenträgers im Gehäuse in einem Teilschnitt entlang der Linie AA von Figur 2.
Die **Figuren 4 und 5** zeigen verschiedene Varianten von Elektrodenanordnungen.

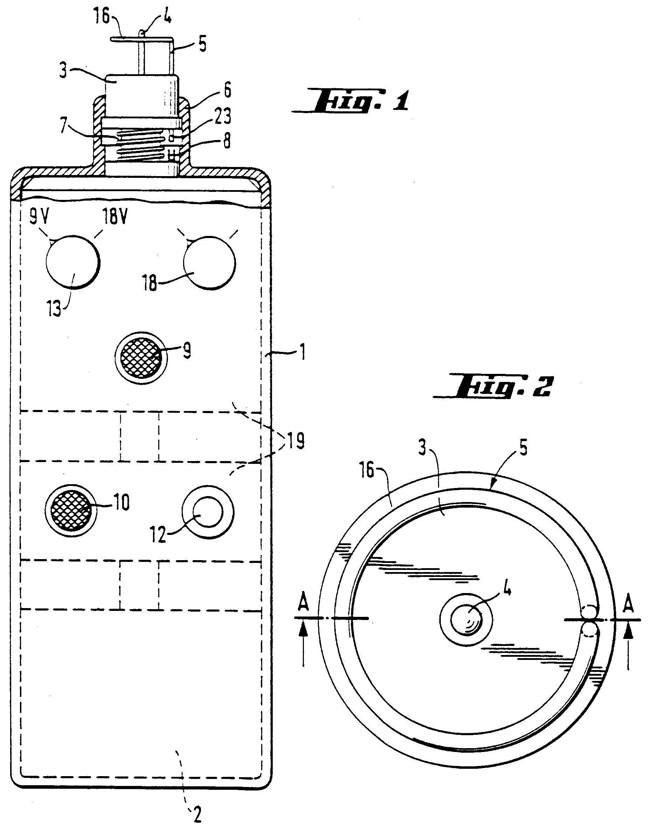

Wie aus Figur 1 ersichtlich, enthält die bevorzugte Ausführungsform der erfindungsgemäßen Vorrichtung ein Gehäuse 1, in dem eine Gleichstromquelle 2, wie z.B. eine Batterie, sowie eine Schalt- und Regeleinheit 19 angeordnet ist.

Die Schalt- und Regeleinheit 19 enthält einen Batterieprüfschalter 12 sowie eine Signaleinrichtung 10, die bei Betätigung des Batterieprüfschalters den Ladezustand der Batterie anzeigt. Diese Signaleinrichtung 10 kann entweder eine Kontrolleuchte oder ein akustischer Signalgeber sein.

Weiterhin enthält die Schalt- und Regeleinheit 19 einen Spannungswahlschalter 13 in Form eines Wechselschalters mit dem die Betriebsspannung aur 9 oder 18 Volt vorgewählt werden kann, einen Umpolschalter 18, mit dem die Polarisation der beiden Elektroden 4, 5 vertauscht werden kann, sowie eine Anzeigevorrichtung 9, die entweder eine Kontrolleuchte oder akustischer Signalgeber sein kann und die anspricht, wenn Ström über die beiden Elektroden 4, 5 fließt. Dabei nimmt die Helligkeit der Kontrolleuchte bzw. die Lautstärke des akustischen Signals proportional zum Stromfluß zu.

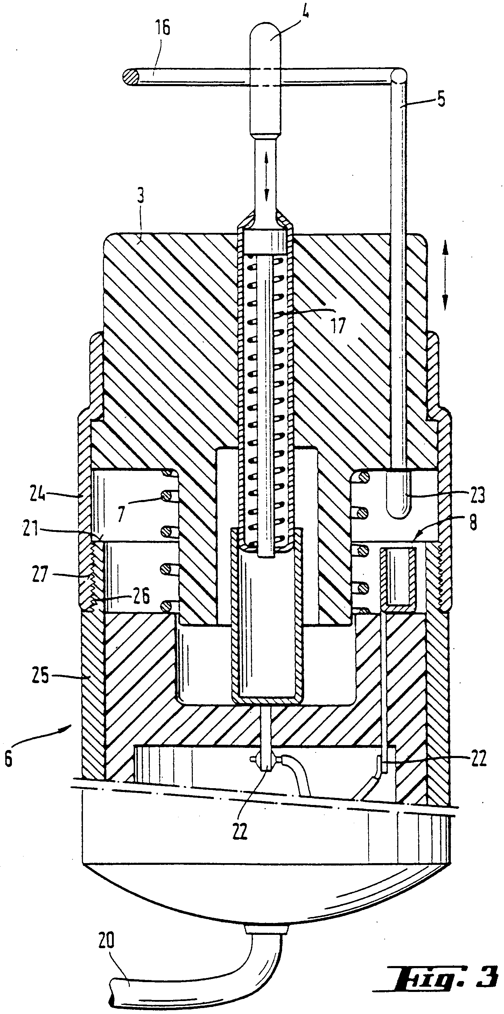

Der Elektrodenträger 3 ist in einem zylindrischen Gehäuseteil 6 geführt und auf einer Feder 7 gelagert. Das zylindrische Gehäuseteil 6 kann entweder, wie in Figur 1 gezeigt, direkt mit dem Gehäuse 1 verbunden sein oder, wie in Figur 3 dargestellt, vom Hauptgehäuse 1 separiert sein und über ein flexibles Anschlußkabel mit der Schalt- und Regeleinheit 19 im Gehäuse 1 verbunden sein.

Wie aus Figur 2 ersichtlich, weist die bevorzugte Ausführungsform zwei Elektroden auf, wobei eine Elektrode 4 aus einem geraden Stift besteht, der zentral im Elektrodenträger 3 angeordnet ist und die dazu parallele Gegenelektrode 5 aus einem Draht besteht, dessen rechtwinklig abgebogenes Teilstück als Drahtring 16 ausgebildet ist, der die erste Elektrode 4 konzentrisch umgibt.

Wie aus Figur 3 ersichtlich, ist die zentrale Elektrode 4 durch den Drahtring 16 der Gegenelektrode 5 hindurchgeführt und im Elektrodenträger 3 federnd gelagert. Dabei ist die Rückstellkraft dieser Feder 17 geringer als die Rückstellkraft der Feder 7, auf der der gesamte Elektrodenträger 3 innerhalb des zylindrischen Gehäuseteils 6 gelagert ist. Die beiden Elektroden 4 und 5 sind über Anschlüsse 22 mit der Schalt- und Regeleinheit 19 des Gehäuses 1 verbunden.

Aus Figur 3 ist auch die Funktionsweise der Vorrichtung ersichtlich. Die Vorrichtung wird mit den Elektroden 4, 5 auf die zu behandelnde Hautstelle aufgelegt. Dabei wird die Elektrode 4 gegen die Rückstellkraft der Feder 17 in den Elektrodenträger 3 hineingedrückt bis sowohl der Drahtring 16 als auch die Spitze der Elektrode 4 auf der zu behandelnden Hautstelle aufliegen.

Die zentrale Elektrode 4 weist dabei vorzugsweise eine Verdickung auf, so daß der Druck der Elektrode auf die eventuell schmerzende Hautstelle gering bleibt. Die federnde Lagerung der Zentralelektrode 4 hat auch den Vorteil, daß Hautunebenheiten ausgeglichen werden können und es stets gewährleistet ist, daß sowohl die Spitze der Elektrode 4 als auch der Drahtring 16 der Gegenelektrode 5 fest aufliegen.

Durch stärkeres Aufdrücken der Elektroden wird der Elektrodenträger 3 gegen die Rückstellkraft der Feder 7 in das zylindrische Gehäuse 6 hineingeschoben, bis er auf den Anschlägen 21 aufliegt. Dabei greift der Schaltstift 23 in den Schalter 8 ein, wodurch der Stromkreis zu den Elektroden geschlossen wird und der Behandlungsstrom zwischen beiden Elektroden fließt.

Zum einfachen Auswechseln des Elektrodenträgers 3 ist das zylindrische Gehäuseteil 6 in ein Deckelteil 24 und ein Bodenteil 25 unterteilt, die jeweils mit korrespondierenden Gewinden 26, 27 versehen und miteinander verschraubt sind.

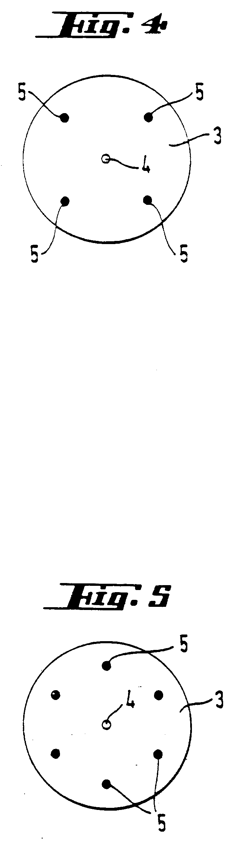

Figur 4 und 5 zeigen alternative Elektrodenanordnungen, wobei jeweils eine federnd im Elektrodenträger 3 gelagerte stiftförmige Zentralelektrode 4 von 4 bzw. 6 ebenfalls stiftförmigen Gegenelektroden 5 umgeben ist.

### BEZUGSZIFFERN

- 1: Gehäuse
- 2: Gleichstromquelle
- 3: Elektrodenträger
- 4: Elektrode
- 5: Elektrode
- 6: zylindrisches Gehäuseteil
- 7: Feder
- 8: Schalter
- 9: Kontrolleuchte
- 10: Kontrolleuchte
- 12: Batterieprüfschalter
- 13: Spannungswahlschalter
- 16: Drahtring
- 17: Feder
- 18: Schalter
- 19: Schalt- und Regeleinheit
- 20: Kabel
- 21: Anschlag
- 22: Anschlüsse
- 23: Schaltstift
- 24: Deckelteil
- 25: Bodenteil
- 26: Gewinde
- 27: Gewinde

## Patentansprüche

1. Eine Vorrichtung zur Behandlung von entzündlichen, sich im Anfangsstadium befindlichen Hautveränderungen, die ein Gehäuse (1) mit darin untergebrachter Gleichstromquelle (2) und einen Elektrodenträger (3), auf dem mindestens zwei entgegengesetzt gepolte Elektroden (4,5) angeordnet sind, die elektrisch mit der Gleichstromquelle (2) verbunden sind, enthält,
**dadurch gekennzeichnet**,
daß eine stiftförmige Elektrode (4) von einer oder mehreren Gegenelektroden (5) konzentrisch umgeben ist, wobei der Elektrodenträger (3) in einem zylindrischen Gehäuseteil (6) beweglich auf einer Feder (7) gelagert ist und die innere Stiftelektrode (4) innerhalb des Elektrodenträgers (3) auf einer Feder (17) gelagert ist, deren Rückstellkraft geringer ist als die der ersten Feder (7), so daß die Elektrode (4) und die Gegenelektrode(n) (5) im Betriebszustand der Vorrichtung auf der zu behandelden Hautregion aufliegen.

2. Vorrichtung nach Anspruch 1
**dadurch gekennzeichnet**,
daß die Gleichstromquelle eine Batterie (2) ist und das Gehäuse (1) zusätzlich eine Schalt- und Regeleinheit (19) mit Kontrolleinrichtungen (9, 10, 11, 12) zur Überprüfung des Ladezustandes der Batterie (2) und des Betriebszustandes der Vorrichtung sowie einer Einstelleinrichtung (13) für die Betriebsspannung enthält.

3. Vorrichtung nach Anspruch 2
**dadurch gekennzeichnet**,
daß die Einstelleinrichtung für die Betriebsspannung ein Wechselschalter (13) ist, mit dem die Betriebsspannung auf 9 oder 18 V vorgewählt werden kann.

4. Vorrichtung nach Anspruch 2
**dadurch gekennzeichnet**,
daß die Einstelleinrichtung eine stufenlose Einstellung der Betriebsspannung zwischen 3 und 18 V ermöglicht.

5. Vorrichtung nach Anspruch 3 oder 4
**dadurch gekennzeichnet**,
daß die Elektroden (4,5) aus korrosionsbeständigem Stahl, Edelmetallen oder aus Legierungen aus Edelmetallen bestehen.

6. Vorrichtung nach Anspruch 5
**dadurch gekennzeichnet**,
daß zu der inneren Stiftelektrode (4) eine Gegenelektrode (6) paralell angeordnete ist, die aus einem Draht besteht, dessen rechtwinklig abgebogenes Teilstück als Drahtschleife (16) ausgebildet ist, die die innere Stiftelektrode (4) konzentrisch umgibt.

7. Vorrichtung nach Anspruch 5
**dadurch gekennzeichnet**,
daß die innere Stiftelektrode (4) von einer zylindrischen Gegenelektrode (5) konzentrisch umgeben ist.

8. Vorrichtung nach Anspruch 5
**dadurch gekennzeichnet**,
daß die innere Stiftelektrode (4) von 3-8 stiftförmigen Gegenelektroden (5), die die Ecken eines Polygons bilden, konzentrisch umgeben ist.

9. Vorrichtung nach Anspruch 6
**dadurch gekennzeichnet**,
daß die Drahtschleife (16) die Form eines Kreises, einer Ellipse oder eines Polygons mit der Eckenzahl 3 - 6 hat.

10. Vorrichtung nach Anspruch 9
**dadurch gekennzeichnet**,
daß der Kreis oder ein Außenkreis, der die Ellipse oder das Polygon umschließt, einen Durchmesser von 5 - 15 mm hat.

11. Vorrichtung nach Anspruch 7
**dadurch gekennzeichnet**,
daß die zylindrische Elektrode (5) einen Durchmesser von 5-15 mm aufweist.

12. Vorrichtung nach Anspruch 8
**dadurch gekennzeichnet**,
daß der Abstand zwischen den äußeren Stiftelektroden (5) und der inneren Stiftelektrode (4) 5 - 15 mm beträgt.

13. Vorichtung nach einem der Ansprüche 10, 11 und 12
**dadurch gekennzeichnet**,
daß die Stiftelektroden (4,5) einen Durchmesser von 0,5 - 2 mm haben.

14. Vorrichtung nach Anspruch 13
**dadurch gekennzeichnet**,
daß der Elektrodenträger (3) gegen die Rückstellkraft der Feder (7) in das zylindrische Gehäuseteil (6) einschiebbar ist, wodurch ein Schalter (8), der den Stromkreis zu den Elektroden (4,5) schließt, betätigt wird.

15. Vorrichtung nach Anspruch 14
**dadurch gekennzeichnet**,
daß zum leichten Auswechseln des Elektrodenträgers (3) das zylindrische Gehäuseteil (6) in ein Deckelteil (24) und ein Bodenteil (25) unterteilt ist, die jeweils mit korrespondierenden Gewinden (26, 27) versehen und miteinander verschraubt sind.

16. Vorrichtung nach Anspruch 15
**dadurch gekennzeichnet**,
daß die Schalt- und Regeleinheit (19) zusätzlich einen Schalter (18) zur Umpolung der Elektroden (4,5) enthält.

17. Vorrichtung nach Anspruch 16
**dadurch gekennzeichnet**,
daß das zylindrische Gehäuseteil (6) in dem Gehäuse (1) integriert ist.

18. Vorrichtung nach nach Anspruch 16
**dadurch gekennzeichnet**,
daß das zylindrische Gehäuseteil (6) griffelförmig ausgebildet, und über ein flexibles Kabel (20) mit dem Gehäuse (1) elektrisch verbunden ist.

## Claims

1. A device for treating inflammatory skin changes in the initial stage, which said device comprises a housing (1), with a direct-current source (2) fitted therein, and an electrode carrier (3) on which are arranged at least two electrodes (4, 5) of opposite polarity, which electrodes are connected electrically to the direct-current source (2), characterized in that a pin-shaped electrode (4) is concentrically surrounded by one or more counter-electrodes (5), the electrode carrier (3) being mounted movably on a spring (7) in a cylindrical housing part (6), and the inner pin electrode (4) being mounted on a spring (17) inside the electrode carrier (3), the restoring force of which spring (17) is less than that of the first spring (7), so that the electrode (4) and the counter-electrode(s) (5) in the operating state of the device bear on the area of skin which is to be treated.

2. Device according to Claim 1, characterized in that the direct-current source is a battery (2) and the housing (1) additionally comprises a switch and control unit (19) with indicator facilities (9, 10, 11, 12) for checking the charge state of the battery (2) and the operating state of the device, and also an adjustment facility (13) for the operating voltage.

3. Device according to Claim 2, characterized in that the adjustment facility for the operating voltage is a change-over switch (13) with which the operating voltage can be preset to 9 or 18 V.

4. Device according to Claim 2, characterized in that the adjustment facility permits a continuous adjustment of the operating voltage between 3 and 18 V.

5. Device according to Claim 3 or 4, characterized in that the electrodes (4, 5) consist of corrosion-resistant steel, noble metals or of alloys of noble metals.

6. Device according to Claim 5, characterized in that
a counter-electrode (6) [sic] is arranged parallel to the inner pin electrode (4) and consists of a wire whose part bent at a right-angle is designed as a wire loop (16) which concentrically surrounds the inner pin electrode (4).

7. Device according to Claim 5, characterized in that the inner pin electrode (4) is concentrically surrounded by a cylindrical counter-electrode (5).

8. Device according to Claim 5, characterized in that the inner pin electrode (4) is concentrically surrounded by 3-8 pin-shaped counter-electrodes (5) which form the corners of a polygon.

9. Device according to Claim 6, characterized in that the wire loop (16) has the shape of a circle, an ellipse or a polygon having corners numbering 3-6.

10. Device according to Claim 9, characterized in that the circle or an outer circle which encloses the ellipse or the polygon has a diameter of 5 - 15 mm.

11. Device according to Claim 7, characterized in that the cylindrical electrode (5) has a diameter of 5 - 15mm.

12. Device according to Claim 8, characterized in that the distance between the outer pin electrodes (5) and the inner pin electrode (4) is 5 - 15 mm.

13. Device according to one of Claims 10, 11 and 12, characterized in that the pin electrodes (4, 5) have a diameter of 0.5-2 mm.

14. Device according to Claim 13, characterized in that the electrode carrier (3) can be pushed into the cylindrical housing part (6) counter to the restoring force of the spring (7), as a result of which a switch (8) which closes the current to the electrodes (4, 5) is activated.

15. Device according to Claim 14, characterized in that, for simple replacement of the electrode carrier (3), the cylindrical housing part (6) is subdivided into a top part (24) and a bottom part (25) which are provided in each case with corresponding threads (26, 27) and are screwed together.

16. Device according to Claim 15, characterized in that the switch and control unit (19) additionally comprises a switch (18) for pole changing of the electrodes (4, 5).

17. Device according to Claim 16, characterized in that the cylindrical housing part (6) is integrated in the housing (1).

18. Device according according [sic] to Claim 16, characterized in that the cylindrical housing part (6) is of pen-shaped design and is connected electrically to the housing (1) via a flexible cable (20).

## Revendications

1. Dispositif pour le traitement d'affections inflammatoires de la peau se trouvant au stade initial, qui comprend un boîtier (1) avec une source de courant continu (2) logée dans celui-ci et un support d'électrodes (3) sur lequel sont disposées au moins deux électrodes polarisées de signes contraires (4, 5), qui sont reliées électriquement à la source de courant continu (2), caractérisé par le fait qu'une électrode en forme de barreau (4) est entourée d'une ou plusieurs contre-électrodes (5) de manière concentrique, le support d'électrodes (3) étant disposé dans une partie cylindrique (6) du boîtier, mobile sur un ressort (7) et l'électrode intérieur en forme de barreau (4) étant placée à l'intérieur du support d'électrodes (3) sur un ressort (17), dont la force de rappel est plus faible que celle du premier ressort (7), de sorte que l'électrode (4) et la ou les contre-électrodes (5) soient placées sur la zone de peau à traiter pendant l'état de fonctionnement du dispositif.

2. Dispositif selon la revendication 1, caractérisé en ce que la source de courant continu est un accumulateur (2) et le boîtier (1) comprend en plus une unité de réglage et de commutation (19) avec des appareils de contrôle (9, 10, 12) pour la vérification de l'état de charge de l'accumulateur (2) et de l'état de fonctionnement du dispositif ainsi qu'un dispositif de réglage (13) de la tension de fonctionnement.

3. Dispositif selon la revendication 2, caractérisé en ce que le dispositif de réglage de la tension de fonctionnement est un commutateur (13), avec lequel on peut présélectionner la tension de fonctionnement à 9 ou 18 volts.

4. Dispositif selon la revendication 2, caractérisé en ce que le dispositif de réglage permet un réglage sans palier de la tension de fonctionnement entre 3 et 18 volts.

5. Dispositif selon l'une des revendications 3 ou 4, caractérisé en ce que les électrodes (4, 5) sont constituées d'acier résistant à la corrosion, de métaux nobles ou d'alliages de métaux nobles.

6. Dispositif selon la revendication 5, caractérisé en ce que, de manière parallèle à l'électrode intérieure en forme de barreau (4), est disposée une contre-électrode (5) qui est constituée d'un fil, dont la partie pliée à angle droit est sous forme de boucle de fil (16), qui entoure de manière concentrique l'électrode intérieure en forme de barreau (4).

7. Dispositif selon la revendication 5, caractérisé en ce que l'électrode intérieure en forme de barreau (4) est entourée de manière concentrique d'une contre-électrode cylindrique (5).

8. Dispositif selon la revendication 5, caractérisé en ce que l'électrode intérieure en forme de barreau (4) est entourée de façon concentrique de 3 à 8 contre-électrodes (5) en forme de barreau, qui forment les sommets d'un polygone.

9. Dispositif selon la revendication 6, caractérisé en ce que la boucle de fil a la forme d'un cercle, d'une ellipse, ou d'un polygone avec un nombre de côtés de 3 à 6.

10. Dispositif selon la revendication 9, caractérisé en ce que le cercle ou un cercle circonscrit, qui entoure l'ellipse ou le polygone a un diamètre de 5 à 15 mm.

11. Dispositif selon la revendication 7, caractérisé en ce que l'électrode cylindrique (5) présente un diamètre de 5 à 15 mm.

12. Dispositif selon la revendication 8, caractérisé en ce que la distance entre les électrodes extérieures en forme de barreau (5) et l'électrode intérieure en forme de barreau (4) est de 5 à 15 mm.

13. Dispositif selon l'une des revendications 10, 11 et 12, caractérisé en ce que les électrodes en forme de barreau (4, 5) ont un diamètre de 0,5 à 2 mm.

14. Dispositif selon la revendication 13, caractérisé en ce que le support d'électrodes (3) est susceptible d'être introduit dans la partie cylindrique (6) du boitier contre la force de rappel du ressort (7), grâce à quoi est actionné un commutateur (8) qui ferme le circuit vers les électrodes (4, 5).

15. Dispositif selon la revendication 14, caractérisé en ce que, afin de faciliter le remplacement du support d'électrodes (3), la partie cylindrique (6) du boîtier est divisée en un couvercle (24) et une boîte (25), qui sont munis chacun de filetages correspondants (26, 27) et qui sont vissés l'un à l'autre.

16. Dispositif selon la revendication 15, caractérisé en ce que l'unité de réglage et de commutation (19) comprend en outre un commutateur (18) pour l'inversion de polarisation des électrodes (4, 5).

17. Dispositif selon la revendication 16, caractérisé en ce que la partie cylindrique (6) du boîtier est incorporée dans le boîtier (1).

18. Dispositif selon la revendication 16, caractérisé en ce que la partie cylindrique (6) du boîtier présente une forme de crayon et est reliée électriquement par un câble flexible (20) au boitier (1).
